(19) Europäisches Patentamt
European Patent Office
Office européen des brevets

(11) Veröffentlichungsnummer: **0 115 248**
**B1**

(12) # EUROPÄISCHE PATENTSCHRIFT

(45) Veröffentlichungstag der Patentschrifft: **28.02.90**

(21) Anmeldenummer: **83730120.9**

(22) Anmeldetag: **12.12.83**

(51) Int. Cl.$^5$: **C 07 D 487/04**, C 07 F 9/40, A 61 K 31/505 // (C07D487/04, 239:00, 209:00)

(54) **Substituierte 5H-Pyrimido(5,4-b)indole.**

(30) Priorität: **16.12.82 DE 3246932**

(43) Veröffentlichungstag der Anmeldung: **08.08.84 Patentblatt 84/32**

(45) Bekanntmachung des Hinweises auf die Patenterteilung: **28.02.90 Patentblatt 90/09**

(84) Bennante Vertragsstaaten: **AT BE CH DE FR GB IT LI LU NL SE**

(56) Entgegenhaltungen:

**CHEMICAL ABSTRACTS, Band 94, Nr. 15, 13. April 1981, Seite 692, Nr. 121446z, Columbus, Ohio, US Z.D. DUBOVENKO u.a.: "Pyrimidines. 72. Synthesis and some properties of 5-amino-2-R-4,6-diphenylpyrimidines and their reaction products"**

**CHEMICAL ABSTRACTS, Band 96, Nr. 11, 15 März 1982, Seite 585, Nr. 85492s, Columbus, Ohio, US N.N. SUVOROV u.a.: "Synthesis and antineoplastic activity of delta-carboline with alkyl and aryl groups in the pyridine ring and of 8H-(4,5-b)indolopyrimidine"**

**Die Akte enthält technische Angaben, die nach dem Eingang der Anmeldung eingereicht wurden und die nicht in dieser Patentschrift enthalten sind.**

(73) Patentinhaber: **SCHERING AKTIENGESELLSCHAFT Berlin und Bergkamen
Müllerstrasse 170/178 Postfach 65 03 11
D-1000 Berlin 65 (DE)**

(72) Erfinder: **Rahtz, Dieter, Dr.
Krottnaurer Strasse 24a
D-1000 Berlin 38 (DE)**
Erfinder: **Huth, Andreas, Dr.
Kyllmannstrasse 15
D-1000 Berlin 39 (DE)**
Erfinder: **Schmiechen, Ralph, Dr.
Bayernring 27
D-1000 Berlin 42 (DE)**
Erfinder: **Seidelmann, Dieter, Dr.
Stierstrasse 14
D-1000 Berlin 41 (DE)**
Erfinder: **Kehr, Wolfgang, Dr.
Biedermannweg 11
D-1000 Berlin 19 (DE)**
Erfinder: **Schneider, Herbert Hans, Dr.
Duisburger Strasse 20
D-1000 Berlin 15 (DE)**
Erfinder: **Braestrup, Claus Thyco, Dr.
Frederiksborgvej 78
DK-4000 Roskilde (DK)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 115 248 B1

LIBERGRAF, STOCKHOLM 1990

**Beschreibung**

Die Erfindung betrifft neue substituierte 5H-Pyrimido[5,4-b]indole gemäß den Ansprüchen 1 - 14 pharmazeutische Präparationen gemäß Anspruch 11 und Verfahren zur Herstellung der neuen 5H-Pyrimido[5,4-b]indole gemäß den Ansprüche 12 - 14.

Die erfindungsgemäßen Verbindungen besitzen wertvolle pharmakologische Eigenschaften. Sie beeinflussen insbesondere das Zentralnervensystem und eignen sich zum Einsatz in psychopharmazeutischen Präparationen.

4-Phenylpyrimidoindole sind aus CA 94 (1981), 121 446z bekannt und in CA 96 (1982), 84 492s wird die Herstellung von 8H-[4,5-b]-indolo-pyrimidin beschrieben.

Die neuen 5H-Pyrimido[5,4-b]indole besitzen die allgemeine Formel I

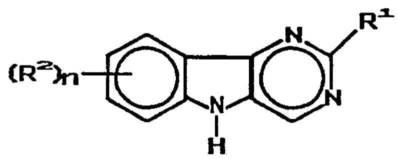

(I)

worin

```
       O - N
      /
R1 = -      ||          mit R3 = C1-3-Alkyl
      \\
       N - C - R3
```

oder $COOR^4$ mit $R^4$ = Wasserstoff oder $C_{1-5}$ Alkyl,

```
                                          O - N
                                         /
R2 = Wasserstoff, Halogen, Nitro, -       ||        (R3 = C1-3-Alkyl),
                                         \\
                                          N - C - R3
```

$OR^4$, $S R^4$,

```
O
||
C-OR4 mit R4 = Wasserstoff, C1-5-Alkyl, C7-10-Aralkyl oder
```

```
O   O
||  ||
C-Cl, C-NR6R7, SO2NR6R7 oder NR6R7 mit R6 und R7 = Wasserstoff, C1-5-Alkyl oder C3-5-Alkenyl
```

und wobei n = 1 oder 2 und $R^2$ in 8- und/oder 9-Stellung stehen kann.

Unter Halogen sind im Rahmen der vorliegenden Erfindung, wenn nichts anderes vermerkt ist, Fluor, Chlor, Brom und Jod zu verstehen.

Unter $C_{1-5}$ Alkyl sind gerad- und verzweigtkettige Gruppen mit bis zu 5 Kohlenstoffatomen zu verstehen, und unter $C_{3-5}$-Alkenyl sollen solche mit 3 bis 5 Kohlenstoffatomen insbesondere Allyl, verstanden werden.

$C_{5-10}$-Aryl soll gruppen mit 5 bis 10 Kohlenstoffatomen, beispielsweise Phenyl, und $C_{7-10}$-Aralkyl mit 7 bis 10 Kohlenstoffatomen, beispielsweise Benzyl und Styryl, einschließen.

Es ist bekannt, daß bestimmte Stellen im zentralen Nervensystem von Vertebraten eine hohe spezifische Affinität für die Bindung von 1,4- und 1,5-Benzodiazepinen aufweisen (Squires, R.F. und Braestrup, C., Nature (London) 266 (1977) 734). Die Stellen werden Benzodiazepin-Rezeptoren genannt.

Es wurde gefunden, daß die erfindungsgemäßen 5H-Pyrimido-[5,4-b]indole, obwohl sie sich in ihrer chemischen Struktur von den Benzodiazepinen unterscheiden, überraschenderweise eine starke Affinität für die Bindung an Benzodiazepin-Rezeptoren zeigen, indem sie radioaktivmarkiertes Flunitrazepam von den Benzodiazepin-Rezeptoren verdrängen.

Die erfindungsgemäßen Verbindungen besitzen ein ähnliches Wirkungsspektrum wie Flunitrazepam. Sie können zu psychopharmazeutischen Präparationen, zum Beispiel für die orale und parenterale Anwendung, formuliert und in der gleichen Weise wie Flunitrazepam angewendet werden.

Als Formulierungshilfsstoffe sind physiologisch verträgliche organische und anorganische Trägersubstanzen geeignet die gegenüber den erfindungsgemäßen Verbindungen inert sind.

Als Trägersubstanzen seien beispielsweise genannt Wasser, Salzlösungen, Alkohole, Polyäthylenglykole polyhydroxyethoxyliertes Rizinusöl, Gelatine, Lactose, Amylose, Magnesiumstearat, Talkum, Kieselsäure, Fettsäuremono- und diglyceride, Pentaerythritolfettsäureester, Hydroxymethylcellulose und Polyvinylpyrrolidon.

Die pharmazeutischen Präparationen können sterilisiert und/oder mit Hilfsstoffen, wie Schmiermitteln, Konservierungsstoffen, Stabilisatoren, Netzmittel, Emulgatoren, Puffermittel und Farbstoffen, versetzt werden.

Für die parenterale Anwendung sind insbesondere Injektionslösungen oder Suspensionen, insbesondere wäßrige Lösungen der aktiven Verbindungen in polyhydroxyethoxyliertem Rizinusöl, geeignet.

Für die orale Anwendung sind insbesondere Tabletten, Dragees oder Kapseln mit Talkum und/oder einem Kohlenwasserstoffträger oder -binder, wie zum Beispiel Lactose, Mais- oder Kartoffelstärke, geeignet. Die Anwendung kann auch in flüssiger Form erfolgen, wie zum Beispiel als Saft, dem gegebenenfalls ein Süßstoff beigefügt wird.

Die erfindungsgemäßen Verbindungen werden in einer Dosiseinheit von 0,05 bis 10 mg aktiver Substanz in einem physiologisch verträglichen Träger eingebracht.

Die erfindungsgemäßen Verbindungen werden in einer Dosis von 0,1 bis 300 mg/Tag, vorzugsweise 1 - 30 mg/Tag, angewendet.

Die Herstellung der Verbindungen der allgemeinen Formel I erfolgt nach klassischen Methoden der organischen Chemie, dadurch, daß man 5-R'-Verbindungen der allgemeinen Formel II

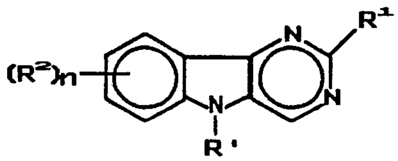

(II)

worin R' eine Tosyl-, Phenylsulfonyl- oder Methoxyphenylsulfonylgruppe, n, $R^2$ und $R^1$ die in Formel I angegebene Bedeutung haben, mit einer starken Base R abspaltet und gegebenenfalls durch Hydrolyse entstandene Säuren gewünschtenfalls verestert und den so erhaltenen Ester gewünschtenfalls in an sich bekannter Weise umestert oder das Amin gegebenenfalls alkyliert oder alkenyliert oder das Amin in einer Sandmeyer-Reaktion mit verdünnter Schwefelsäure, mit Halogenwasserstoffsäure in Gegenwart von Kupfer(I)-halogenid, mit $R^4$-OH oder $(R^4)_2S_2$, wobei $R^4$ die oben angegebene Bedeutung besitzt, umsetzt und gewünschtenfalls eine $R^4$O-Verbindung einer Etherspaltung unterwirft und gewünschtenfalls die OH-Verbindung erneut verethert oder gegebenfalls eine freie Säure mit einem Amidoxim $R^3$-C(=NOH)$NH_2$, mit $R^3$ = $C_{1-3}$-Alkyl, zum Oxadiazolylderivat umsetzt.

Das Verfahren zur Herstellung von Verbindungen der allgemeinen Formel I mit $R^1$ in der Bedeutung von $COOR^{4'}$ ist dadurch gekennzeichnet, daß man eine Verbindung der allgemeinen Formel III

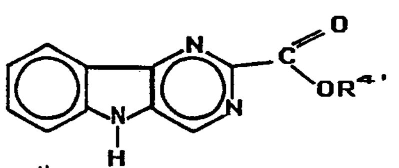

(III)

worin $R^{4'}$ die in Formel I angegebene Bedeutung hat, in an sich bekannter Weise nitriert und gegebenenfalls die erhaltenen Nitroverbindungen zu den entsprechenden Aminoverbindungen reduziert und gegebenenfalls die Aminoverbindungen in o-Stellung halogeniert oder das Amin alkyliert, alkenyliert oder in einer Sandmeyer-Reaktion mit verdünnter Schwefelsäure, mit Halogenwasserstoffsäure in Gegenwart von Kupfer(I)-halogenid, mit $R^4$OH oder $(R^4)_2S_2$, wobei $R^4$ die oben angegebene Bedeutung besitzt, umsetzt oder in an sich bekannter Weise halogeniert und das so erhaltene Halogenierungsprodukt gegebenenfalls zur $(R^5O)_2OP$-, $R^4O$- oder $R^4OCO$-Verbindung umsetzt, verseift oder amidiert und gegebenenfalls die Hydroxycarbonylverbindung zur Halogencarbonylverbindung umsetzt, die Halogencarbonylverbindung gegebenenfalls mit einem Amin der Formel $R^6R^7NH$ oder einem Alkohol der Formel $R^4OH$ umsetzt oder in an sich bekannter Weise sulfonyliert und gegebenenfalls die so erhaltenen Chlorsulfonylverbindungen mit einem Amin der Formel $R^6R^7NH$ umsetzt.

Das Verfahren zur Herstellung von Verbindungen der allgemeinen Formel I mit $R^1$ in der Bedeutung von

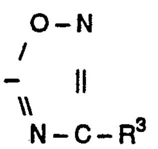

ist dadurch gekennzeichnet, daß man eine Verbindung der allgemeinen Formel IV

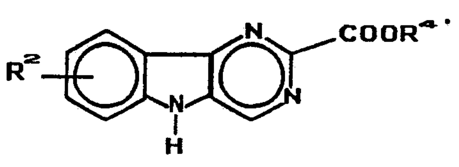

(IV)

worin
$R^2$ für Wasserstoff, $NR^6R^7$ oder $COOR^4$, wobei $R^{4'}$, $R^4$, $R^6$ und $R^7$ die oben angegebenen Bedeutungen haben, steht, gewünschtenfalls in an sich bekannter Weise hydrolisiert und die so erhaltene freie Saure mit einer Verbindung der Formel $R^3$-C(=NOH)$NH_2$, mit $R^3$ = $C_{1-3}$ Alkyl zum Oxadiazolyiderivat umsetzt.

Aus 5-R'-Verbindungen der allgemeinen Formel II wird in an sich bekannter Weise R' abgespalten. Je nach der Bedeutung von $R^2$ und $R^1$ im Endprodukt der Formel I können die vorhandenen Substituenten $R^2$ und $R^1$ abgewandelt oder ein oder mehrere Substituenten $R^1$ eingeführt und gewünschtenfalls anschließend abgewandelt werden.

Die Abspaltung der Gruppe R' gemäß Anspruch 12 wird mit starken Basen vorgenommen. Geeignete Basen sind zum Beispiel Alkalialkoholate und Alkalihydroxide in alkoholischer Lösung. Die Umsetzung wird durch längeres Stehen bei Raumtemperatur oder durch Erhitzen unter Rückfluß bewirkt.

Bei Vorliegen einer Estergruppe und bei der Alkalibehandlung in der Siedehitze wird der Ester teilweise hydrolisiert und kann gewünschtenfalls durch Behandlung mit dem entsprechenden Alkohol in Gegenwart einer Säure zurückgebildet werden.

Eine gegebenenfalls gewünschte Umesterung der Estergruppe erfolgt ebenfalls nach an sich bekannten Methoden mit dem Alkohol $R^4OH$, beispielsweise in Gegenwart eines sauren Katalysators wie Schwefelsäure, p-Toluolsulfonsäure, HCl oder $CuCl_2$ in der Siedehitze.

Die Umwandlung der Aminoverbindungen in die entsprechenden Hydroxy- Halogen-, $R^4O$- und $R^4S$-Verbindungen geschieht ebenfalls in an sich bekannter Weise, beispielsweise nach der Sandmeyer-Reaktion, bei der das diazotierte Produkt direkt mit verdünnter Schwefelsäure, mit Halogenwasserstoffsäure in Gegenwart von Kupfer(I)-halogenid, mit $R^4OH$ oder $(R^4)_2S_2$ bei angehobenen Temperaturen umgesetzt wird.

Ether ($R^4O$)-Verbindungen der allgemeinen Formel I können in an sich bekannter Weise zu den Hydroxy($R^{40}$)-Verbindungen hydrolysiert werden. Die Hydrolyse wird vorzugsweise mit Lewis-Säuren wie zum Beispiel Bortribromid in Methylenchlorid oder Bromwasserstoffsäure in Wasser, durch längeres Stehen bei Raumtemperatur oder durch Erhitzen bei Rückflußtemperatur bewirkt.

Eine gegebenenfalls gewünschte Veretherung der Hydroxygruppe in den Verbindungen der allgemeinen Formel I erfolgt ebenfalls nach bekannten Methoden. Hierzu wird die Hydroxyverbindung zum Beispiel in Gegenwart von Alkalicarbonat in einem polaren Lösungsmittel, wie Acetonitril, Dimethylformamid oder 1-Methyl-2-pyrrolidon, mit Alkyl-, Arylalkyl- oder Arylhalogenid umgesetzt.

Das Nitrieren der Verbindungen der allgemeinen Formel III gemäß Anspruch 13 wird ebenfalls nach an sich bekannten Verfahren durchgeführt. So kann das Ausgangsmaterial bei Temperaturen zwischen 0 und 100°C mit konzentrierter Salpetersäure oder einem Gemisch aus konzentrierter Salpetersäure und konzentrierter Schwefelsäure umgesetzt werden. Die bei dem Nitrieren verwendete Säure dient dabei sowohl als Reagenz als auch als Lösungsmittel. Mit 65-%-iger Salpetersäure wird eine Nitrogruppe, vorzugsweise in 6- oder 8-Stellung, eingeführt.

Die sich gegebenenfalls anschließende Reduktion der erhaltenen Nitroverbindung zur entsprechenden Aminoverbindung erfolgt gleichfalls nach an sich bekannten Methoden.

Eine bevorzugte Methode ist die Reduktion mit Wasserstoff in Gegenwart von Metallkatalysatoren wie Raney-Nickel, Platin in feinverteilter Form oder Palladium auf einem geeigneten Träger wie Kohle oder Kalk bei Normaldruck und Raumtemperatur. Möglich ist aber auch die Verwendung von Wasserstoff zu statu nascendi, zum Beispiel durch Zink/Salzsäure.

Eine so erhaltene 8-Aminoverbindung kann mit Halogenwasserstoffsäure in einem oxidierenden Lösungsmittel wie Dimethylsulfoxid in 9-Stellung halogeniert werden. Aus der 8-Aminoverbindung wird zum Beispiel mit Bromwasserstoffsäure die 8-Amino-9-brom-Verbindung erhalten. Gewünschtenfalls kann die Aminogruppe anschließend in an sich bekannter Weise eliminiert werden.

Zur Herstellung von Verbindung der allgemeinen Formel I mit einer substituierten Aminogruppe $NR^6R^7$ werden entsprechende mit einer Aminogruppe ($R^2$)substituierte 5H-Pyrimido[5,4-b]indol-2-carbonsäureester nach an sich bekannten Methoden in einem geeigneten Lösungsmittel in Gegenwart einer Base mit einem Alkyl- oder Alkenyl-, halogenid, -tosylat oder -mesylat bei Temperaturen im Bereich von Raumtemperatur bis zum Siedepunkt des Reaktionsgemisches, vorzugsweise bei 20 bis 120°C, umgesetzt.

Für die Umsetzung geeignet sind an sich alle protischen und aprotischen Lösungsmittel, soweit sie gegenüber den Reaktanten inert sind. Beispielsweise seien genannt aliphatische Alkohole wie Methanol, Ethanol und Propanol, Ketone wie Aceton und Methylisobutylketon, Ether wie Glykoldimethylether und Diethylether, cyclische Ether wie Tetrahydrofuran und Dioxan, sowie Lösungsmittel wie Dimethylformamid, Dimethylacetamid und N-Methylpyrrolidon.

Als Basen sind an sich alle starken organischen Basen wie Triethylamin, 4-Dimethylaminopyridin, Ethylendiisopropylamin, Diazabicyclo-undecan, -nonan und -octan geeignet. Möglich ist aber auch die Verwendung eines Alkalimetallcarbonats wie Natrium- oder Kaliumcarbonat, aber auch von Alkoholaten wie Kalium-tert.butylat.

Die Umwandlung der Aminogruppe $R^2$ in die entsprechende Hydroxy-, Halogen-, $R^4O$- und $R^4S$-Verbindungen geschieht wiederum in an sich bekannter Weise mit Hilfe der Sandmeyer-Reaktion.

Die Halogenierung der Verbindungen der allgemeinen Formel III gemäß Anspruch 13 erfolgt nach an sich bekannten Methoden. Hierzu wird das Ausgangsmaterial in einem inerten Lösungsmittel gelöst und mit dem entsprechenden Halogen wie Chlor oder Brom gegebenenfalls in Gegenwart eines basischen Katalysators bei Temperaturen zwischen 0 und 50°C umgesetzt. Inerte Lösungsmittel sind beispielsweise chlorierte Kohlenwasserstoffe wie Methylenchlorid, Chloroform, Dichlorethylen usw. Als basische Katalysatoren eignen sich Pyridin und substituierte Pyridine wie 4-Dimethylaminopyridin. Ein basischer Katalysator ist bei der Chlorierung entbehrlich.

Zur Einführung von Jod verwendet man zweckmäßigerweise nicht nur elementares Jod, sondern ein Gemisch von Jod und Jodsäure, wobei die Reaktion vorzugsweise in Eisessig bei 80 bis 100°C unter Protonenkatalyse durchgeführt wird.

Die sich gegebenenfalls anschließende Umsetzung der Jodverbindung mit Dialkylphosphit wird in Gegenwart eines löslichen Edelmetallkomplexes, wie zum Beispiel Palladiumtetrakis(triphenylphosphin), und einer Base, wie Triethylamin, Pyridin oder 4-Dimethylaminopyridin, in einem aprotischen polaren Lösungsmittel bei Temperaturen von 20 bis 140°C durchgeführt. Als aprotische polare Lösungsmittel kommen zum Beispiel Hexamethylphosphorsäuretriamid und N-Methylpyrrolidon infrage.

Die Halogenverbindung, insbesondere die 8-Jodverbindung, kann außerdem mit Palladium(II)-acetat und Kohlenmonoxid in Alkoholen, wie zum Beispiel Benzylalkohol, und in Gegenwart eines tert. Amins, wie zum Beispiel Triethylamin, Tributylamin oder Pyridin, carbonyliert werden. Die so erhaltene 8-Benzyloxycarbonylverbindung kann anschließend in bekannter Weise verseift oder mit einem Amin der Formel $R^6R^7NH$ zur Reaktion gebracht werden. Die 8-Benzyloxycarbonylgruppe kann aber auch durch Hydrierung selektiv debenzyliert werden.

Die freie Hydroxycarbonylverbindung kann in an sich bekannter Weise in die Halogencarbonylverbindung, beispielsweise mit Thionylchlorid in die Chlorcarbonylverbindung überführt werden.

Die Halogencarbonylverbindung kann wiederum in bekannter Weise mit einem Amin der Formel $R^6R^7NH$ oder einem Alkohol der Formel $R^4OH$ umgesetzt werden.

Die Sulfochlorierung der Verbindungen der allgemeinen Formel III gemäß Anspruch 13 erfolgt nach an sich bekannten Methoden. Hierzu wird das Ausgangsmaterial gegebenenfalls in einem inerten Lösungsmittel wie Methylenchlorid oder Chloroform mit Chlorsulfonsäure versetzt. Zur Herstellung von entsprechenden Aminsulfonsäure-Derivaten wird das so erhaltene Produkt mit einem Amin der Formel $R^6R^7NH$ zur Reaktion gebracht und anschließend auf 60 bis 100°C erwärmt.

Stellt der Substituent $R^1$ und/oder $R^2$ in Formel I einen Oxadiazolylrest dar, so geht man von einer Verbindung der Formel IV aus, in welcher $R^2$ und/oder $R^1$ die Bedeutung eines Esters hat. Der Ester wird nach an sich bekannten Methoden verseift z. B. mit wässrigem Alkalihydroxid in der Wärme. Anschließend wird die freie Carbonsäure mit einem Amidoxim der Formel $R^3$-C(=NOH)$NH_2$, wobei $R^3$ einen niederen Alkylrest mit 1 - 3 Kohlenstoffatomen darstellt, in einem Lösungsmittel, das über 100°C siedet und gegenüber den Reaktanten inert ist, bei der Rückflußtemperatur des Reaktionsgemisches zur Kondensation gebracht. Geeignete Lösungsmittel für die Kondensationsreaktion sind beispielsweise Toluol und Dimethylformamid. Zweckmäßigerweise wird die freie Carbonsäure vor der Kondensationsreaktion in geeigneter Weise aktiviert. Möglich ist z. B. die Überführung der Carbonsäure in das gemischte Anhydrid, in den aktivierten Ester oder in das Chlorid. Gut bewährt hat sich eine Aktivierung mit Imidazol/Thionylchlorid in einem aprotischen Lösungsmittel wie Dioxan Tetrahydrofuran, Dimethylformamid oder N-Methylpyrrolidon bei Temperaturen zwischen 0 und 50°C, vorzugsweise bei Raumtemperatur.

**Beispiel 1**

**5H-Pyrimido[5,4-b]indol-2-carbonsäure**

Zu einer Lösung von 2,36 g 5-Tosyl-5H-pyrimido[5,4-b]indol-2-carbonsäure-ethylester in 70 ml Ethanol wird in einer Argonatmosphäre eine Lösung von 0,355 g Natrium in 35 ml Ethanol getropft. Anschließend wird eine halbe Stunde am Rückfluß gekocht. Der nach dem Erkalten auskristallisierende Niederschlag wird abgesaugt und aus Wasser umkristallisiert. Ausbeute 0,12 g 5H-Pyrimido[5,4-b]indol-2-carbonsäure vom Schmelzpunkt 265 - 268°C. Aus der Ethanolmutterlauge werden durch Eindampfen und Extraktion mit Essigester 0,3 g 5H-Pyrimido[5,4-b]indol-2-carbonsäure-ethylester (siehe Beispiel 2) isoliert.

Der als Ausgangsmaterial benötigte 5-Tosyl-5H-pyrimido[5,4-b]indol-2-carbonsäure-ethylester wird folgendermaßen hergestellt:

a) Zu 5 g 3-Amino-1-tosylindol-2-carbonitril werden langsam unter Rühren und Eiskühlung 10 ml Oxalsäureethylesterchlorid getropft. Das klumpige Reaktionsgemisch wird durch Rühren mit Ethanol fein verteilt und abgesaugt. Die Ausbeute beträgt 4,8 g Oxalsäuremonoethylester-mono-(2-cyano-1-tosyl-3-indolyl)amid. Schmelzpunkt: 156 - 157°C.

b) 3,2 g Oxalsäuremonoethylester-mono-(2-cyano-1-tosyl-3-indolyl)amid werden in 480 ml Ethanol unter Zusatz von 6 g Raney-Ni bei Raumtemperatur unter Normaldruck hydriert. Die Dauer der Wasserstoffaufnahme beträgt etwa 20 Stunden.

Die vom Katalysator abfiltrierte Lösung wird eingedampft und der Rückstand mit einem Gemisch gleicher Teile Toluol und Essigester über Kieselgel chromatographiert. So werden 0,4 g 5-Tosyl-5H-pyrimido[5,4-b]indol-2-carbonsäure-ethylester und 0,2 g 1,4-Dihydro-5-tosyl-5n-pyrimido[5,4-b]indol-2-carbonsäure-ethylester vom Schmelzpunkt 175 - 178°C erhalten.

c) 10 g 1,4-Dihydro-5-tosyl-5H-pyrimido[5,4-b]indol-2-carbonsäure-ethylester werden unter Argon in 200 ml Xylol mit 5 g 10 %-iger Pd-Kohle 10 Stunden am Rückfluß gekocht. Anschließend wird das Filtrat von der Palladiumkohle eingedampft, der Eindampfrückstand ergibt nach Umkristallisieren aus einem Essigester/Ethergemisch 5 g 5-Tosyl-5H-pyrimido[5,4-b]indol-2-carbonsäure-ethylester vom Schmelzpunkt 187 - 189°C.

**Beispiel 2**

**5H-Pyrimido[5,4-b]indol-2-carbonsäure-ethylester**
12 g 5H-Pyrimido[5,4-b]indol-2-carbonsäure (Beispiel 1) werden in 50 ml Ethanol suspendiert und unter Zusatz von 1,1 ml konzentrierter Schwefelsäure in einer Argonatmosphäre 8 Stunden am Rückfluß gekocht. Anschließend wird das Reaktionsgemisch eingeengt und der Rückstand in Methylenchlord/Wasser aufgenommen. Die Methylenchloridphase wird mit Wasser gewaschen, getrocknet und eingedampft. Der Rückstand wird aus Ethanol umkristallisiert.
Ausbeute: 1,1 g 5H-Pyrimido[5,4-b]indol-2-carbonsäureethylester vom Schmelzpunkt 244 - 246°C.

**Beispiel 3**

**5H-Pyrimido[5,4-b]indol-2-carbonsäure-n-propylester**
0,5 g 5H-Pyrimido[5,4-b]indol-2-carbonsäure-ethylester (Beispiel 2) werden in 5 ml n-Propanol mit einer Spatelspitze p-Toluolsulfonsäure 45 Stunden am Rückfluß gekocht. Das Reaktionsgemisch wird anschließend eingedampft und unter Behandlung mit Aktivkohle aus Isopropanol umkristallisiert.
Ausbeute: 0,15 g 5H-Pyrimido[5,4-b]indol-2-carbonsäure-n -propylester vom Schmelzpunkt 222 - 224°C.

**Beispiel 4**

**8-Nitro-5H-pyrimido[5,4-b]indol-2-carbonsäure-ethylester**
0,325 g 5H-Pyrimido[5,4-b]indol-2-carbonsäure-ethylester (Beispiel 2) werden in 6 ml 65 %-iger Salpetersäure 15 Minuten bei Raumtemperatur gerührt, danach 1 Stunde bei 70°C. Danach wird das Reaktionsgemisch in Eiswasser gegossen und mit Essigester extrahiert. Die vereinigten Essigesterextrakte werden eingedampft, der Eindampfrückstand wird an Kieselgel mit einem Gemisch aus gleichen Teilen Methylenchlorid und Aceton chromatographiert. So werden 0,023 g 8-Nitro-5H-pyrimido[5,4-b]indol-2-carbonsäure-ethylester vom Schmelzpunkt 304 - 306°C erhalten.

**Beispiel 5**

**8-Amino-5H-pyrimido[5,4-b]indol-2-carbonsäure-ethylester**
1,5 g 8-Nitro-5H-pyrimido[5,4-b]indol-2-carbonsäure-ethylester (Beispiel 4) werden in 90 ml eines Gemisches gleicher Teile Tetrahydrofuran und Methanol unter Zusatz von 0,21 g 10-%-iger Palladiumkohle bei Normaltemperatur unter einem Wasserstoffdruck von 70 bar hydriert. Die vom Katalysator befreite Lösung wird eingedampft, der Rückstand wird in Essigester und wenig Ethanol aufgenommen, filtriert und zu 3/4 eingedampft. Es fallen 0,400 g 8-Amino-5H-pyrimido[5,4-b]indol-2-carbonsäure-ethylester vom Schmelzpunkt 256 - 258°C aus.

**Beispiel 6**

**8-Diallylamino-5H-pyrimido[5,4-b]indol-2-carbonsäure-ethylester**
0,4 g 8-Amino-5H-pyrimido[5,4-b]indol-2-carbonsäure-ethylester (Beispiel 5) werden in 8 ml Ethanol mit 0,16 ml Diazabicycloundecan und 0,25 ml Allylbromid unter Argon 5 Stunden bei 50 - 60°C gerührt. Anschließend wird die Reaktionslösung eingedampft, und der Rückstand wird in Essigester aufgenommen. Diese Lösung wird erst mit Wasser, dann mit Natriumbicarbonatlösung und schließlich nochmals mit Wasser gewaschen, mit Natriumsulfat getrocknet und eingeengt. Man erhält 0,18 g 8-Diallylamino-5H-pyrimido[5,4-b]indol-2-carbonsäure-ethylester vom Schmelzpunkt 188 - 190°C.

**Beispiel 7**

**8-Jod-5H-pyrimido[5,4-b]indol-2-carbonsäure-ethylester**
2 g 5H-Pyrimido[5,4-b]indol-2-carbonsäure-ethylester (Beispiel 2) werden in 20 ml Eisessig mit 0,5 ml Wasser. 0,12 ml konzentrierter Schwefelsäure. 336 mg Jodsäure und 878 mg Jod versetzt und 2,5 Stunden bei 90°C gerührt. Nach Einengen wird mit 50 ml Wasser versetzt, vorsichtig mit Ammoniak alkalisch gestellt und abgesaugt. Man erhält 2.81 g 8-Jod-5H-pyrimido[5,4-b]indol-2-carbonsäure-ethylester vom Schmelzpunkt 264 - 266°C unter Zersetzung.

**Beispiel 8**

**8-Brom-5H-pyrimido[5,4-b]indol-2-carbonsäure-ethylester**
1,15 g 5H-Pyrimido[5,4-b]indol-2-carbonsäure-ethylester (Beispiel 2) werden in 50 ml Chloroform und 5,3 ml Pyridin gelöst und mit 1 g (0,33 ml) Brom in 10 ml Chloroform bei Raumtemperatur tropfenweise versetzt. Nach 6 Stunden Rühren wird eingeengt, in 30 ml Wasser aufgenommen, mit Ammoniak alkalisch gestellt und mit Essigester überschichtet, geschüttelt und abgesaugt. Als Rückstand erhält man 0,86 g 8-Brom-5H-pyrimido[5,4-b]indol-2-carbonsäure-ethyle-

ster vom Schmelzpunkt 286 - 290°C (unter Zersetzung).

**Beispiel 9**

**8-N,N-Dimethylsulfonamido-5H-pyrimido[5,4-b]indol-2-carbonsäure-ethylester**
486 mg 5H-Pyrimido[5,4-b]indol-2-carbonsäure-ethylester (Beispiel 2) werden langsam bei 4°C in 2 ml Chlorsulfonsäure eingetragen. Anschließend wird vorsichtig auf 60°C erwärmt und 30 Minuten bei dieser Temperatur weitergerührt. Nach dem Abkühlen wird auf 25 ml Eis getropft (heftige Reaktion). Man neutralisiert dann mit 40-%-iger wäßriger Dimethylaminlösung. Es wird darauf vom unlöslichen Rest abgesaugt und das Filtrat dreimal mit je 50 ml Essigester ausgeschüttelt. Die organische Phase wird einmal mit gesättigter Kochsalzlösung gewaschen, mit Calciumsulfat getrocknet, filtriert und eingeengt. Den Rückstand chromatographiert man über Kieselgel mit Methylenchlorid/Ethanol = 10 : 1 als Elutionsmittel und erhält 105 mg 8-N,N-Dimethylsulfonamdio-5H-pyrimido[5,4-b]indol-2-carbonsäure-ethylester vom Schmelzpunkt 299 - 301°C unter Zersetzung.

**Beispiel 10**

**8-Diethylphosphoryl-5H-pyrimido[5,4-b]indol-2-carbonsäure-ethylester**
510 mg 8-Jod-5H-pyrimido[5,4-b]indol-2-carbonsäure-ethylester (Beispiel 7) werden zu einem Gemisch von 0,22 ml Diethylphosphit, 0,22 ml Triethylamin, 120 mg Palladiumtetrakis-(triphenylphosphin) in 25 ml N-Methylpyrrolidon gegeben und unter Argon 4 Stunden bei 90°C gerührt. Nach Zugabe von 0,22 ml Diethylphosphit, 0,22 ml Triethylamin und 120 mg Palladiumtetrakistriphenylphosphin wird nochmals 3 Stunden auf 90°C erhitzt. Nach Einengen bei 80°C Badtemperatur (Ölpumpe) wird der Rückstand über Kieselgel unter Methylenchlorid/Ethanol = 10 : 1 als Elutionsmittel chromatographiert. Nach Umkristallisation der entsprechenden Fraktionen aus Essigester/Diisopropylether erhält man 131 mg 8-Diethylphosphoryl-5H-pyrimido[5,4-b]indol-2-carbonsäure-ethylester vom Schmelzpunkt 195 - 197°C.

**Beispiel 11**

**8-Benzyloxycarbonyl-5H-pyrimido[5,4-b]indol-2-carbonsäure-ethylester**
700 mg 8-Jod-5H-pyrimido[5,4-b]indol-2-carbonsäure-ethylester (Beispiel 7) werden in 12 ml Benzylalkohol mit 0,5 ml Tri(n)butylamin unter Kohlenmonoxidatmosphäre auf 110°C erwärmt, mit 20 mg Palladium(II)-acetat versetzt und 3 Stunden bei 110°C gerührt. Nach Abdestillieren des Benzylalkohols im Ölpumpenvakuum wird in Dimethylformamid und Ethanol gelöst und vom Katalysator abgesaugt. Anschließend wird eingeengt, mit Ethanol ausgerührt und abgesaugt. Man erhält 501 mg 8-Benzyloxycarbonyl-5H-pyrimido[5,4-b]indol-2-carbonsäure-ethylester vom Schmelzpunkt 271 - 272°C.

**Beispiel 12**

**8-Hydroxycarbonyl-5H-pyrimido[5,4-b]indol-2-carbonsäure-ethylester**
1,18 g 8-Benzyloxycarbonyl-5H-pyrimido[5,4-b]indol-2-carbonsäure-ethylester (Beispiel 11) werden in einem Gemisch aus 60 ml N-Methylpyrrolidon, 20 ml Methanol und 10 ml 1 n Salzsäure mit 498 mg Palladium auf Kohle (10 %) 2 Stunden bei Raumtemperatur unter einem Bar Wasserstoffdruck hydriert. Nach Absaugen über Kieselgur wird eingeengt. Der Rückstand wird in einem Gemisch aus Essigester, Ethanol und Petrolether ausgerührt, und man erhält 900 mg 8-Hydroxycarbonyl-5H-pyrimido[5,4-b]indol-2-carbonsäure-ethylester in Form eines Hydrochlorids.

**Beispiel 13**

**8-Chlorcarbonyl-5H-pyrimido[5,4-b]indol-2-carbonsäure-ethylester**
184 mg 8-Hydroxycarbonyl-5H-pyrimido[5,4-b]indol-2-carbonsäure-ethylester (Beispiel 12) werden in 5 ml Thionylchlorid mit einem Tropfen Dimethylformamid 3 Stunden am Rückfluß gekocht. Nach Einengen wird ohne weitere Reinigung weiterumgesetzt.

**Beispiel 14**

**8-N,N-Dimethylcarbamoyl-5H-pyrimido[5,4-b]indol-2-carbonsäure-ethylester**
180 mg 8-Chlorcarbonyl-5H-pyrimido[5,4-b]indol-2-carbonsäure-ethylester (Beispiel 13) werden in 5 ml absolutem Tetrahydrofuran suspendiert und bei 4°C tropfenweise mit einer 1molaren Lösung von Dimethylamin in Tetrahydrofuran bis pH8 versetzt. Anschließend wird 2 Stunden bei 4°C gerührt. Nach Eintragen in Wasser wird abgesaugt. Der Rückstand wird in Essigester/Ethanol ausgerührt, und man erhält 90 mg 8-N,N-Dimethylcarbamoyl-5H-pyrimido[5,4-b]indol-2-carbonsäure-ethylester vom Schmelzpunkt 289 - 292°C (unter Zersetzung).

**Beispiel 15**

**8-N,N-Diallylcarbamoyl-5H-pyrimido[5,4-b]indol-2-carbonsäure-ethylester**
Analog Beispiel 14 wird die Titelverbindung hergestellt.

**Beispiel 16**

**8-Ethoxycarbonyl-5H-pyrimido[5,4-b]indol-2-carbonsäure-ethylester**
Zu einer Suspension von 150 mg 8-Chlorcarbonyl-5H-pyrimido[5,4-b]indol-2-carbonsäure-ethylester (Beispiel 13) in 5 ml Tetrahydrofuran werden mit einer Lösung von Triethylamin in Ethanol bei 4°C bis zum pH 8 versetzt. Anschließend wird 2 Stunden bei Raumtemperatur gerührt und eingedampft. Nach Verteilen in Essigester/verdünnter Ammoniaklösung wird die organische Phase abgetrennt, getrocknet, filtriert und eingeengt. Nach Chromatographie über Kieselgel mit Methylenchlorid/Ethanol = 10 : 1 als Elutionsmittel erhält man 70 mg 8-Ethoxycarbonyl-5H-pyrimido[5,4-b]indol-2-carbonsäure-ethylester.

**Beispiel 17**

**8-Methylthio-5H-pyrimido[5,4-b]indol-2-carbonsäure-ethylester**
400 mg 8-Amino-5H-pyrimido[5,4-b]indol-2-carbonsäure-ethylester (Beispiel 5) werden in 4,8 ml Dimethyldisulfid unter Argon auf 60°C erwärmt. Anschließend wird mit 2,8 ml Isoamylnitrit versetzt und 1 Stunde auf 80°C erwärmt. Nach Eindampfen wird über Kieselgel mit Methylenchlorid/Ethanol = 10 : 1 als Elutionsmittel chromatographiert. Nach Umkristallisation der entsprechenden Fraktionen erhält man 130 mg 8-Methylthio-5H-pyrimido[5,4-b]indol-2-carbonsäure-ethylester vom Schmelzpunkt 265 - 266°C.

**Beispiel 18**

**8-Amino-9-brom-5H-pyrimido[5,4-b]indol-2-carbonsäure-ethylester**
1 g 8-Amino-5H-pyrimido[5,4-b]indol-2-carbonsäure-ethylester (Beispiel 5) wird mit so viel Dimethylsulfoxid versetzt, daß eine klare Lösung entsteht. Dann wird bei 0 - 5°C die gleiche Menge 48 %-iger wäßriger Bromwasserstoffsäure zugetropft und danach weitere 2 Stunden bei dieser Temperatur gerührt. Man erhält 8-Amino-9-brom-5H-pyrimido[5,4-b]indol-2-carbonsäure-ethylester.

**Beispiel 19**

**9-Brom-5H-pyrimido[5,4-b]indol-2-carbonsäure-ethylester**
Zu einer Suspension von 1 g 8-Amino-9-brom-5H-pyrimido-[5,4-b]indol-2-carbonsäure-ethylester in 70 ml Ethanol werden 1,8 ml Isoamylnitrit gefügt. Das Gemisch wird 1 Stunde am Rückfluß gekocht und anschließend eingeengt.
Ausbeute: 0,2 g 9-Brom-5H-pyrimido[5,4-b]indol-2-carbonsäure-ethylester.

**Beispiel 20**

**9-Methoxy-5H-pyrimido[5,4-b]indol-2-carbonsäure-ethylester**
Die Verbindung wird, ausgehend von 3-Amino-4-methoxy-1-tosylindol-2-carbonitril, nach dem in den Beispielen 1 und 2 geschilderten Verfahren hergestellt.
Das als Ausgangsmaterial benötigte 3-Amino-4-methoxy-1-tosylindol-2-carbonitril wird aus 2-Amino-6-methoxybenzonitril analog zur literaturbekannten Synthese des 3-Amino-1-tosyl-indol-2-carbonitril (Kenneth Clarke, William Richard Fox und Richard M. Scrowston: J. Chem. Res. 1980 (2) 833 - 847) gewonnen.

**Beispiel 21**

**9-Hydroxy-5H-pyrimido[5,4-b]indol-2-carbonsäure-ethylester**
Zu einer Suspension von 0,52 g 9-Methoxy-5H-pyrimido-[5,4-b]indol-2-carbonsäure-ethylester (Beispiel 20) in 50 ml Methylenchlorid werden 4 ml einer 1-molaren Lösung von Bortribromid in Methylenchlorid gefügt. Das Reaktionsgemisch wird über Nacht unter Argon gerührt. Anschließend werden unter Kühlung mit Eiswasser 2 ml Ethanol zum Reaktionsgemisch getropft. Nach Zugabe von 500 ml Ether wird der Niederschlag von 9-Hydroxy-5H-pyrimido[5,4-b]indol-2-carbonsäure-ethylester abgesaugt.

**Beispiel 22**

**9-Benzyloxy-5H-pyrimido[5,4-b]indol-2-carbonsäure-ethylester**

In einer Argonatmosphäre wird eine Lösung von 0,2 g 9-Hydroxy-5H-pyrimido[5,4-b]indol-2-carbonsäure-ethylester (Beispiel 21) in 20 ml Dimethylformamid mit 0,2 g Kaliumcarbonat 20 Minuten gerührt. Anschließend werden 0.18 g Benzylchlorid zugesetzt. Das Gemisch wird weiter unter Argon über Nacht gerührt. Dann wird es im Vakuum so weit wie möglich eingedampft. Der Rückstand wird mit Wasser ausgerührt. Der wasserunlösliche Anteil ergibt nach Chromatographie über Kieselgel (Laufmittel Methylenchlorid Ethanol = 10 : 1) den gewünschten 9-Benzyloxy-5H-pyrimido[5,4-b]indol-2-carbonsäure-ethylester.

**Beispiel 23**

**8-Diallylamino-2-(3-ethyl-1.2.4-oxadiazol-5-yl)-5H-pyrimido[5,4-b]indol**

150 mg 8-Diallylamino-5H-pyrimido(5,4-b)-indol-2-carbonsäure-ethylester werden in 25 ml Ethanol und 5 ml Wasser mit 200 mg Kalilauge 1 Stunde am Rückfluß gekocht. Nach Einengen, Zufügen von Wasser und Eisessig wird abgesaugt und getrocknet. Die so erhaltene freie Säure (130 mg) wird in 10 ml trockenem Tetrahydrofuran mit 1,5 mMol (6 ml) einer frisch bereiteten Lösung von Thionylimidazol in Tetrahydrofuran versetzt. Nach Reaktion der Säure wird 400 mg Propionamidoxim zugefügt. Nachdem das Säureimidazolid über Nacht reagiert hat, wird eingedampft, in Wasser und Eisessig aufgenommen, filtriert und luftgetrocknet. Es wird dann in 20 ml Toluol aufgenommen und 2 Stunden am Rückfluß gekocht. Nach Filtrieren und Einengen erhält man 62 mg 8-Diallylamino-2-(3-ethyl-1.2.4-oxadiazol-5-yl)-5H-pyrimido(5,4-b)-indol vom Schmelzpunkt 75 - 184°C.

In analoger Weise werden hergestellt:

2-(3-Ethyl-1.2.4-oxadiazol-5-yl)-5H-pyrimido(5,4-b)-indol, F. 250 - 257°C aus
5H-Pyrimido-(5,4-b)-indol-2-carbonsäureethylester und
2.8-Di-(3-ethyl-1.2.4-oxadiazol-5-yl)-5H-pyrimido(5,4-b)-indol F. 244 - 257°C,
aus 8-Ethoxycarbonyl-5H-pyrimido(5,4-b)-indol-2-carbonsäure-ethylester und der verdoppelten Menge an Reagenzien.

**Patentansprüche**

1. Substituierte 5H-Pyrimido[5,4-b]indole der allgemeinen Formel I

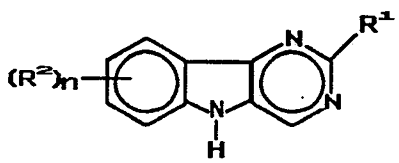

(I)

worin

```
          O – N
         /
R¹ = –       ||        mit R³= C1-3-Alkyl
         \\
          N – C – R³
```

oder-COOR$^{4'}$ mit $R^{4'}$= Wasserstoff oder -$C_{1-5}$-Alkyl,

```
                                   O – N
                                  /
R² = Wasserstoff, Halogen, Nitro, –       ||        (R³= C1-3-Alkyl),
                                  \\
                                   N – C – R³
```

–$OR^4$,-S $R^4$,

```
O
||
–C-OR⁴
```
mit $R^4$ = Wasserstoff, $C_{1-5}$-Alkyl, $C_{7-10}$-Aralkyl oder $C_{5-10}$-Aryl, -PO($OR^5$)$_2$ mit $R^5$ = $C_{1-5}$-Alkyl,

$-\overset{O}{\overset{\|}{C}}-Cl$, $-\overset{O}{\overset{\|}{C}}-NR^6R^7$, $-SO_2NR^6R^7$ oder $-NR^6R^7$ mit $R^6$ und $R^7$= Wasserstoff, $C_{1-5}$-Alkyl oder $C_{3-5}$-Alkenyl und wobei n = 1 oder 2 und $R^2$ in 8- und/oder 9-Stellung stehen kann.

2. Substituierte 5H-Pyrimido[5,4-b]indole der allgemeinen Formel I, gemäß Anspruch 1 wobei $R^2$ in 8-Stellung steht.

3. Substituierte 5H-Pyrimido[5,4-b]indole der allgemeinen Formel I, gemäß Anspruch 1 wobei $R^2$ in 9-Stellung steht.

4. Substituierte 5H-Pyrimido[5,4-b]indole der allgemeinen Formel I, gemäß Anspruch 1 wobei $R^2$ in 8- und 9-Stellung steht.

5. Substituierte 5H-Pyrimido[5.4-b]indole der allgemeinen Formel I, gemäß Anspruch 1 wobei $R^1$ und/oder $R^2$ für einen Oxadiazolylring steht.

6. 8-Diallylamino-2-(3-ethyl-1.2.4-oxadiazol-5-yl)-5H-pyrimido[5,4-b]-indol
2-(3-Ethyl-1.2.4-oxadiazol-5-yl)-H-pyrimido[5,4-b]-indol
2.8-Di-(3-ethyl-1.2.4-oxadiazol-5-yl)-5H-pyrimido-[5,4-b]-indol

7. 5H-Pyrimido[5,4-b]indol-2-carbonsäure
5H-Pyrimido[5,4-b]indol-2-carbonsäure-ethylester
5H-Pyrimido[5,4-b]indol-2-carbonsäure-n-propylester

8. 8-Nitro-5H-pyrimido[5,4-b]indol-2-carbonsäure-ethylester
8-Amino-5H-pyrimido[5,4-b]indol-2-carbonsäure-ethylester
8-Diallylamino-5H-pyrimido[5,4-b]indol-2-carbonsäure-ethylester
8-Amino-9-brom-5H-pyrimido[5,4-]indol-2-carbonsäure-ethylester

9. 9-Brom-5H-pyrimido[5,4-b]indol-2-carbonsäure-ethylester
9-Methoxy-5H-pyrimido[5,4-b]indol-2-carbonsäure-ethylester
9-Hydroxy-5H-pyrimido[5,4-b]indol-2-carbonsäure-ethylester
9-Benzyloxy-5H-pyrimido[5,4-b]indol-2-carbonsäure-ethylester

10. 8-Jod-5H-pyrimido[5,4-b]indol-2-carbonsäure-ethylester
8-Brom-5H-pyrimido[5,4-b]indol-2-carbonsäure-ethylester
8-N,N-Dimethylsulfonamido-5H-pyrimido[5,4-b]indol-2-carbonsäure-ethylester
8-Diethylphosphoryl-5H-pyrimido[5,4-b]indol-2-carbonsäure-ethylester
8-Benzyloxycarbonyl-5H-pyrimido[5,4-b]indol-2-carbonsäure-ethylester
8-Hydroxycarbonyl-5H-pyrimido[5,4-b]indol-2-carbonsäure-ethylester
8-Chlorcarbonyl-5H-pyrimido[5,4-b]indol-2-carbonsäure-ethylester
8-N,N-Dimethylcarbamoyl-5H-pyrimido[5,4-b]indol-2-carbonsäure-ethylester
8-N,N-Diallylcarbamoyl-5H-pyrimido[5,4-b]indol-2-carbonsäure-ethylester
8-Ethoxycarbonyl-5H-pyrimido[5,4-b]indol-2-carbonsäure-ethylester
8-Methylthio-5H-pyrimido[5,4-b]indol-2-carbonsäure-ethylester

11. Pharmazeutische Präparationen, gekennzeichnet durch den Gehalt an einer Verbindung der Ansprüche 1 bis 10.

12. Verfahren zur Herstellung vom Verbindungen der allgemeinen Formel I gemäß Anspruch 1, dadurch gekennzeichnet, daß man 5-R -Verbindungen der allgemeinen Formel II

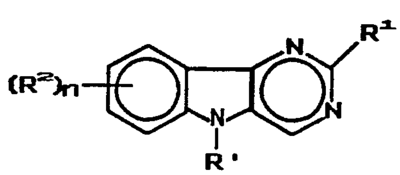

(II)

worin R eine Tosyl-, Phenylsulfonyl- oder Methoxyphenylsulfonylgruppe, n, $R^2$ und $R^1$ die in Formel I gemäß Anspruch 1 angegebene Bedeutung haben, mit einer starken Base R abspaltet und gegebenenfalls durch Hydrolyse entstandene Sauren gewünschtenfalls verestert und den so erhaltenen Ester gewünschtenfalls in an sich bekannter Weise umestert oder das Amin gegebenenfalls alkyliert oder alkenyliert oder das Amin in einer Sandmeyer-Reaktion mit verdünnter Schwefelsäure, mit Halogenwässerstoffsäure in Gegenwart von Kupfer(I)-halogenid, mit $R^4$-OH oder $(R^4)_2S_2$, wobei $R^4$ die oben angegebene Bedeutung besitzt, umsetzt und gewünschtenfalls eine $R^4$O-Verbindung einer Etherspaltung unterwirft und gewünschtenfalls die OH-Verbindung erneut verethert oder gegebenfalls eine freie Säure mit einem Amido-

xim $R^3$-C(=NOH)$NH_2$, mit $R^3$ = $C_{1-3}$-Alkyl, zum Oxadiazolylderivat umsetzt.

13. Verfahren zur Herstellung von Verbindungen der allgemeinen Formel I gemäß Anspruch 1 mit $R^1$ in der Bedeutung von $COOR^{4'}$ dadurch gekennzeichnet, daß man eine Verbindung der allgemeinen Formel III

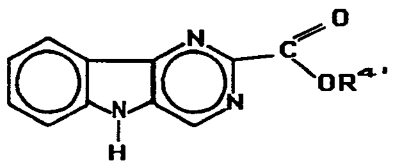

(III)

worin $R^{4'}$ die in Formel I angegebene Bedeutung hat, in an sich bekannter Weise nitriert und gegebenenfalls die erhaltenen Nitroverbindungen zu den entsprechenden Aminoverbindungen reduziert und gegebenenfalls die Aminoverbindungen in o-Stellung halogeniert oder das Amin alkyliert, alkenyliert oder in einer Sandmeyer-Reaktion mit verdünnter Schwefelsaure, mit Halogenwasserstoffsäure in Gegenwart von Kupfer(I)-halogenid, mit $R^4OH$ oder $(R^4)_2S_2$, wobei $R^4$ die oben angegebene Bedeutung besitzt, umsetzt oder in an sich bekannter Weise halogeniert und das so erhaltene Halogenierungsprodukt gegebenenfalls zur $(R^5O)_2OP$-, $R^4$- oder $R^4OCO$-Verbindung umsetzt, verseift oder amidiert und gegebenenfalls die Hydroxycarbonylverbindung zur Halogencarbonylverbindung umsetzt, die Halogencarbonylverbindung gegebenenfalls mit einem Amin der Formel $R^6R^7NH$ oder einem Alkohol der Formel $R^4OH$ umsetzt oder in an sich bekannter Weise sulfonyliert und gegebenenfalls die so erhaltenen Chlorsulfonylverbindungen mit einem Amin der Formel $R^6R^7NH$ umsetzt.

14. Verfahren zur Herstellung von Verbindungen der allgemeinen Formel I mit $R^1$ in der Bedeutung von

```
 O – N
 /
–     ||
 \\
 N – C – R3
```

dadurch gekennzeichnet, daß man eine Verbindung der allgemeinen Formel IV

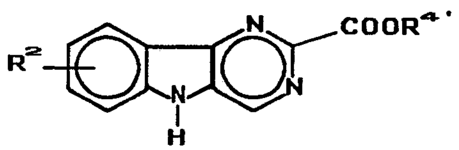

(IV)

worin $R^2$ für Wasserstoff, $NR^6R^7$ oder $COOR^4$ wobei $R^4$, $R^{4'}$, $R^6$ und $R^7$ die oben angegebenen Bedeutungen haben, steht, gewünschtenfalls in an sich bekannter Weise hydrolisiert und die so erhaltene freie Saure mit einer Verbindung der Formel $R^3$-C(=NOH)$NH_2$, mit $R^3$ = $C_{1-3}$Alkyl zum Oxadiazolylderivat umsetzt.

**Claims**

1. Substituted 5H-pyrimido[5,4-b]indoles of the general formula I

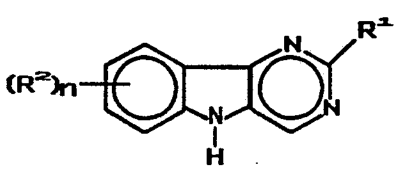

(I)

wherein

```
        O – N
        /
R1 = –     ||        wherein R3 = C1-3-alkyl,
        \\
        N – C – R3
```

or -COOR$^{4'}$ wherein R$^{4'}$ = hydrogen or $C_{1-5}$-alkyl,

```
                              O – N
                             /
R2 = hydrogen, halogen, nitro, –      ||
                             \\
                              N – C – R3
```

($R^3$ = $C_{1-3}$-alkyl), -OR$^{4}$, -SR$^4$

```
O
||
```
-C-OR$^4$ wherein $R^4$ = hydrogen, $C_{1-5}$-alkyl, $C_{7-10}$-aralkyl or $C_{5-10}$-aryl, -PO(OR$^5$)$_2$ wherein $R^5$ = $C_{1-5}$-alkyl,

```
O     O
||    ||
```
-C-Cl, -C-NR$^6$R$^7$, -SO$_2$NR$^6$R$^7$ or -NR$^6$R$^7$ wherein $R^6$ and $R^7$ = hydrogen, $C_{1-5}$-alkyl or $C_{3-5}$-alkenyl, and wherein n = 1 or 2 and $R^2$ may be in the 8- and/or 9-position.

2. Substituted 5H-pyrimido[5,4-b]indoles of the general formula I according to claim 1 wherein $R^2$ is in the 8-position.

3. Substituted 5H-pyrimido[5,4-b]indoles of the general formula I according to claim 1 wherein $R^2$ is in the 9-position.

4. Substituted 5H-pyrimido[5,4-b]indoles of the general formula I according to claim 1 wherein $R^2$ is in the 8- and 9-position.

5. Substituted 5H-pyrimido[5,4-b]indoles of the general formula I according to claim 1 wherein $R^1$ and/or $R^2$ represents an oxadiazolyl ring.

6. 8-Diallylamino-2-(3-ethyl-1,2,4-oxadiazol-5-yl)-5H-pyrimido[5,4-b]indole
2-(3-Ethyl-1,2,4-oxadiaxol-5-yl)-H-pyrimido[5,4-b]-indole
2,8-Di-(3-ethyl-1,2,4-oxadiazol-5-yl)-5H-pyrimido-[5,4-b]-indole

7. 5H-Pyrimido[5,4-b]indole-2-carboxylic acid
5H-Pyrimido[5,4-b]indole-2-carboxylic acid ethyl ester
5H-Pyrimido[5,4-b]indole-2-carboxylic acid n-propyl ester

8. 8-Nitro-5H-pyrimido[5,4-b]indole-2-carboxylic acid ethyl ester
8-Amino-5H-pyrimido[5,4-b]indole-2-carboxylic acid ethyl ester
8-Diallylamino-5H-pyrimido[5,4-b]indole-2-carboxylic acid ethyl ester
8-Amino-9-bromo-5H-pyrimido[5,4-b]indole-2-carboxylic acid ethyl ester

9. 9-Bromo-5H-pyrimido[5,4-b]indole-2-carboxylic acid ethyl ester
9-Methoxy-5H-pyrimido[5,4-b]indole-2-carboxylic acid ethyl ester
9-Hydroxy-5H-pyrimido[5,4-b]indole-2-carboxylic acid ethyl ester
9-Benzyloxy-5H-pyrimido[5,4-b]indole-2-carboxylic acid ethyl ester

10. 8-Iodo-5H-pyrimido[5,4-b]indole-2-carboxylic acid ethyl ester
8-Bromo-5H-pyrimido[5,4-b]indole-2-carboxylic acid ethyl ester
8-N,N-Dimethylsulfonamido-5H-pyrimido[5,4-b]indole-2-carboxylic acid ethyl ester
8-Diethylphosphoryl-5H-pyrimido[5,4-b]indole-2-carboxylic acid ethyl ester
8-Benzyloxycarbonyl-5H-pyrimido[5,4-b]indole-2-carboxylic acid ethyl ester
8-Hydroxycarbonyl-5H-pyrimido[5,4-b]indole-2-carboxylic acid ethyl ester
8-Chlorocarbonyl-5H-pyrimido[5,4-b]indole-2-carboxylic acid ethyl ester
8-N,N-Dimethylcarbamoyl-5H-pyrimido[5,4-b]indole-2-carboxylic acid ethyl ester
8-N,N-Diallylcarbamoyl-5H-pyrimido[5,4-b]indole-2-carboxylic acid ethyl ester
8-Ethoxycarbonyl-5H-pyrimido[5,4-b]indole-2-carboxylic acid ethyl ester
8-Methylthio-5H-pyrimido[5,4-b]indole-2-carboxylic acid ethyl ester

11. Pharmaceutical preparations, characterised by a content of a compound of claims 1 to 10.

12. Process for the preparation of compounds of the general formula I according to claim 1, characterised in that in 5-R' compounds of the general formula II

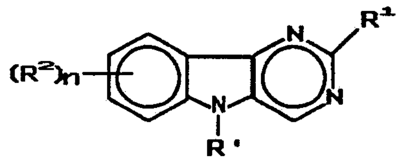

(II)

wherein R' represents a tosyl, phenylsufonyl or methoxyphenylsulfonyl group, and n, $R^2$ and $R^1$ have the meanings given in formula I according to claim 1, R' is split off with a strong base and, if desired, any acids that may have been produced by hydrolysis are esterified and, if desired, the ester so obtained is transesterified in a manner known *per se* or the amine is optionally alkylated or alkenylated or the amine is reacted in a Sandmeyer reaction with dilute sulfuric acid, with hydrohalic acid in the presence of copper(I) halide, with $R^4$-OH or $(R^4)_2S_2$ wherein $R^4$ has the meanings given above, and, if desired, an $R^4O$ compound is subjected to ether cleavage and, if desired, the OH compound is again etherified or optionally a free acid is reacted with an amidoxime $R^3$-C(=NOH)$NH_2$ wherein $R^3$ = $C_{1-3}$-alkyl to form the oxadiazolyl derivative.

13. Process for the preparation of compounds of the general formula I according to claim 1 wherein $R^1$ represents $COOR^{4'}$, characterised in that a compound of the general formula III

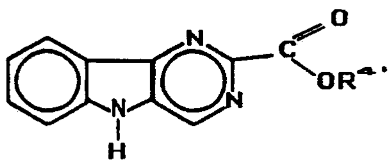

(III)

wherein $R^{4'}$ has the meanings given in formula I according to claim 1, is nitrated in a manner known *per se* and optionally the resulting nitro compounds are reduced to the corresponding amino compounds and optionally the amino compounds are halogenated in the o-position or the amine is alkylated, alkenylated or reacted in a Sandmeyer reaction with dilute sulfuric acid, with hydrohalic acid in the presence of copper(I) halide, with $R^4OH$ or $(R^4)_2S_2$ wherein $R^4$ has the meanings given above, or is halogenated in a manner known *per se* and the halogenation product so obtained is reacted to form the $(R^5O)_2OP$, $R^4O$ or $R^4OCO$ compound, saponified or amidated and optionally the hydroxycarbonyl compound is reacted to form the halocarbonyl compound, the halocarbonyl compound is optionally reacted with an amine of formula $R^6R^7NH$ or an alcohol of formula $R^4OH$, or is sulfonylated in a manner known *per se,* and optionally the chlorosulfonyl compound so obtained is reacted with an amine of the formula $R^6R^7NH$.

14. Process for the preparation of compounds of the general formula I according to claim 1 wherein $R^1$

represents 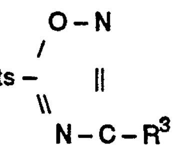

characterised in that a compound of the general formula

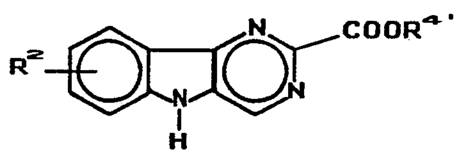

(IV)

wherein $R^2$ represents hydrogen, $NR^6R^7$ or $COOR^4$, and wherein $R^{4'}$, $R^4$, $R^6$ and $R^7$ have the meanings given above, is if desired hydrolysed in a manner known *per se* and the free acid so obtained is reacted with a compound of the formula $R^3$-C(=NOH)$NH_2$ wherein $R^3$ = $C_{1-3}$-alkyl to form the oxadiazolyl derivative.

**Revendications**

1. 5H-Pyrimido[5,4-b]indoles substitués qui répondent à la formule générale I:

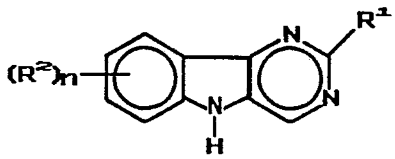

(I)

dans laquelle:

O – N
/
$R^1$ représente un radical –   ||
\\
N – C – $R^3$

dont le substituant $R^3$ désigne un alkyle en $C_1$ - $C_3$, ou représente un radical -$COOR^{4'}$ dans lequel $R^4$ désigne l'hydrogène ou un alkyle en $C_1$ - $C_5$,

$R^2$ représente l'hydrogène, un halogène, un nitro, un

O – N
/
radical –   ||
\\
N – C – $R^3$

(où $R^3$ désigne un alkyle en $C_1$ - $C_3$), un radical -$OR^4$, -$SR^4$ ou -C(=O)-$OR^4$ (où $R^4$ désigne l'hydrogène, un alkyle en $C_1$ - $C_5$, un aralkyle en $C_7$ - $C_{10}$ ou un aryle en $C_5$ - $C_{10}$), un radical -$PO(OR^5)_2$ (où $R^5$ désigne un alkyle en $C_1$ - $C_5$), un radical -C(=O)-Cl ou un radical -C(=O)-$NR^6R^7$, -$SO_2NR^6R^7$ ou -$NR^6R^7$ (où $R^6$ et $R^7$ désignent chacun l'hydrogène, un alkyle en $C_1$ - $C_5$ ou un alcényle en $C_3$ - $C_5$), et n est égal à 1 ou à 2, $R^2$ pouvant se trouver à la position 8 et/ou à la position 9.

2. 5H-Pyrimido[5,4-b]indoles substitués de formule générale I selon la revendication 1, dans lesquels $R^2$ se trouve à la position 8.

3. 5H-Pyrimido[5,4-b]indoles substitués de formule générale I selon la revendication 1, dans lesquels $R^2$ se trouve à la position 9.

4. 5H-Pyrimido[5,4-b]indoles substitués de formule générale I selon la revendication 1, dans lesquels les $R^2$ se trouvent à la position 8 et à la position 9.

5. 5H-Pyrimido[5,4-b]indoles substitués de formule générale I selon la revendication 1, dans lesquels $R^1$ ou $R^2$, ou chacun d'eux, représente un cycle oxadiazolyle.

6. Diallylamino-8 (éthyl-3 oxadiazole-1,2,4 yl-5)-2 5H-pyrimido[5,4-b]indole,
(éthyl-3 oxadiazole-1,2,4 yl-5)-2 5H-pyrimido[5,4-b]indole,
bis-(éthyl-3 oxadiazole-1,2,4 yl-5)-2,8 5H-pyrimido[5,4-b]indole.

7. Acide 5H-pyrimido[5,4-b]indole-carboxylique-2,
ester éthylique de l'acide 5H-pyrimido[5,4-b]indole-carboxylique-2,
ester n-propylique de l'acide 5H-pyrimido[5,4-b]indole-carboxylique-2.

8. Ester éthylique de l'acide nitro-8 5H-pyrimido[5,4-b]indole-carboxylique-2, ester éthylique de l'acide amino-8 5H-pyrimido[5,4-b]indole-carboxylique-2, ester éthylique de l'acide diallylamino-8 5H-pyrimido[5,4-b]indole-carboxylique-2, ester éthylique de l'acide amino-8 bromo-9 5H-pyrimido[5,4-b]indole-carboxylique-2.

9. Ester éthylique de l'acide bromo-9 5H-pyrimido[5,4-b]indole-carboxylique-2, ester éthylique de l'acide méthoxy-9 5H-pyrimido[5,4-b]indole-carboxylique-2, ester éthylique de l'acide hydroxy-9 5H-pyrimido[5,4-b]indole-carboxylique-2, ester éthylique de l'acide benzyloxy-9 5H-pyrimido[5,4-b]indole-carboxylique-2.

10. Ester éthylique de l'acide iodo-8 5H-pyrimido[5,4-b]indole-carboxylique-2, ester éthylique de l'acide bromo-8 5H-pyrimido[5,4-b]indole-carboxylique-2, ester éthylique de l'acide diméthylsulfamoyl-8 5H-pyrimido[5,4-b]indole-carboxylique-2, ester éthylique de l'acide diéthylphosphoryl-8 5H-pyrimido[5,4-b]indole-carboxylique-2, ester éthylique de l'acide benzyloxycarbonyl-8 5H-pyrimido[5,4-b]indole-carboxylique-2, ester éthylique de l'acide hydroxycarbonyl-8 5H-pyrimido[5,4-b]indole-carboxylique-2, ester éthylique de l'acide chlorocarbonyl-8 5H-pyrimido[5,4-b]indole-carboxylique-2, ester éthylique de l'acide diméthylcarbamoyl-8 5H-pyrimido[5,4-b]indole-carboxylique-2, ester éthylique de l'acide diallylcarbamoyl-8 5H-pyrimido[5,4-b]indole-carboxylique-2, ester éthylique de l'acide éthoxycarbonyl-8 5H-pyrimido[5,4-b]indole-carboxylique-2, ester éthylique de l'acide méthylthio-8 5H-pyrimido[5,4-b]indole-carboxylique-2.

11. Compositions pharmaceutiques caractérisées en ce qu'elles contiennent un composé selon l'une quelconque des revendications 1 à 10.

12. Procédé pour préparer des composés de formule générale I selon la revendication 1, procédé caractérisé en ce qu'on élimine le radical R', au moyen d'une base forte, de composés répondant à la formule générale II:

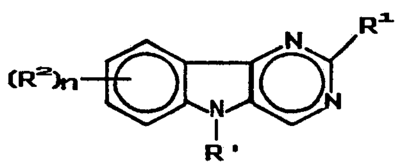

(II)

dans laquelle R' représente un radical tosyle, phénylsulfonyle ou méthoxyphényl-sulfonyle, et n, $R^2$ et $R^1$ ont les significations qui leur ont été données à propos de la formule I dans la revendication 1, et, si on le désire, on estérifie des acides qui ont pu se former par hydrolyse et, si on le désire, on transestérifie de manière connue l'ester ainsi obtenu, ou on alkyle ou alcényle éventuellement l'amine ou on fait réagir l'amine, par une réaction de Sandmeyer avec de l'acide sulfurique dilué, avec un acide halogénhydrique en présence d'un halogénure de cuivre(I), avec $R^4OH$ ou $(R^4)_2S_2$, le symbole $R^4$ ayant la signification indiquée ci-dessus, et, si on le désire, on soumet un composé à radical $R^4O$- à une coupure d'éther et, si on le désire, on éthérifie à nouveau le composé à radical -OH, ou on fait éventuellement réagir un acide libre avec un amide-oxime $R^3$-C(=NOH)$NH_2$, dans lequel $R^3$ désigne un alkyle en $C_1$-$C_3$, de manière à obtenir le composé oxadiazolylique.

13. Procédé de préparation de composés de formule générale I selon la revendication 1 dans lesquels $R^1$ représente un radical -$COOR^{4'}$, procédé caractérisé en ce qu'on nitre de manière connue un composé répondant à la formule générale III:

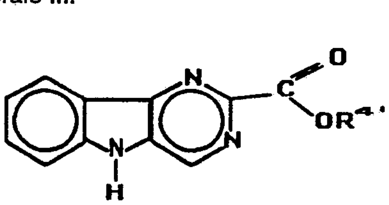

(III)

dans laquelle $R^{4'}$ a la signification qui lui a été donnée à propos de la formule I dans la revendication 1, et on réduit éventuellement les composés nitrés obtenus en les composés aminés correspondants et éventuellement on halogène les composés aminés en position ortho ou on alkyle ou alcényle l'amine ou on la fait réagir, par une réaction de Sandmeyer, avec de l'acide sulfurique dilué, avec un acide halogénhydrique en présence d'un halogénure de cuivre(I), avec $R^4OH$ ou $(R^4)_2S_2$, le symbole $R^4$ ayant la signification indiquée ci-dessus, ou on halogène de manière connue et on fait éventuellement réagir le produit d'halogénation ainsi obtenu pour le convertir en un composé porteur d'un radical $(R^5O)_2OP$-, $R^4O$- ou $R^4OCO$-, on le saponifie ou on l'amidifie, et éventuellement on fait réagir le composé hydroxycarbonylique pour le convertir en le composé halogénocarbonylique et on fait éventuellement réagir le composé halogénocarbonylique avec une amine de formule $R^6R^7NH$ ou un alcool de formule $R^4OH$, ou on sulfonyle de manière connue et on fait éventuellement réagir le composé chlorosulfonylique ainsi obtenu avec une amine de formule $R^6R^7NH$.

14. Procédé de préparation de composés de formule générale I selon la revendication 1 dans lesquels $R^1$ représente un radical:

```
  O – N
 /
–      ||
 \\
  N – C – R3
```

procédé caractérisé en ce qu'on hydrolyse éventuellement, de manière connue, un composé répondant à la formule générale IV:

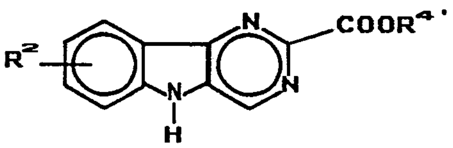

(IV)

dans laquelle:
$R^2$ représente l'hydrogène, un radical $-NR^6R^7$ ou un radical $-COOR^4$, les symboles $R^{4'}$, $R^4$, $R^6$ et $R^7$ ayant les significations indiquées ci-dessus, et on fait réagir l'acide libre ainsi obtenu avec un composé répondant à la formule $R^3$-$C(=NOH)NH_2$ dans laquelle $R^3$ désigne un alkyle en $C_1$-$C_3$, de manière à le convertir en le composé oxadiazolylique.